(19) Europäisches Patentamt
European Patent Office
Office européen des brevets

(11) Publication number: 0 190 686
A2

(12) # EUROPEAN PATENT APPLICATION

(21) Application number: 86101306.8

(22) Date of filing: 31.01.86

(51) Int. Cl.⁴: C 12 N 15/00
C 12 N 1/20
//(C12N1/20, C12R1:19)

(30) Priority: 02.02.85 JP 18991/85

(43) Date of publication of application:
13.08.86 Bulletin 86/33

(84) Designated Contracting States:
BE CH DE FR GB LI NL SE

(71) Applicant: THE GREEN CROSS CORPORATION
15-1, Imabashi-1-chome Higashi-ku Osaka-shi
Osaka 541(JP)

(72) Inventor: Ishida, Yutaka
24-9, Kitanakaburi 1-chome
Hirakata-shi Osaka(JP)

(72) Inventor: Nagai, Masanori
10-10, Nishiyamawaki
Yawata-shi Kyoto(JP)

(72) Inventor: Arimura, Hirofumi
18-1-401, Uenosaka 2-chome
Toyonaka-shi Osaka(JP)

(72) Inventor: Suyama, Tadakazu
3-7, Matsuigaoka 4-chome Tanabe-cho
Tsuzuki-gun Kyoto(JP)

(74) Representative: Barz, Peter, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

(54) **Method of producing interferon-gamma.**

(57) A method of producing interferon-γ which comprises using, as the gene expression elements, a expression elements containing an α-amylase promoter region derived from a gram-positive bacterial strain and, as the host, a gram-negative bacterial strain.

The invention has thus enabled production of interferon-γ in gram-negative bacteria through the use of gene expression elements of a gram-positive bacterial strain and has contributed to technology diversification in the field of genetic engineering. On the occasion of the secretion of the interferon-γ into the periplasmic space of gram-negative bacteria, the signal peptide is cleaved off, whereby the protein is obtained in the form of a mature protein free of methionine at the N terminal.

EP 0 190 686 A2

# METHOD OF PRODUCING INTERFERON-$\gamma$

## FIELD OF THE INVENTION

This invention relates to a method of producing interferon-$\gamma$ by a genetic engineering technique.

## BACKGROUND OF THE INVENTION

Genetic engineering techniques are widely used, for example in the production of useful heterologous proteins.

Escherichia coli, Bacillus subtilis and yeasts, among others, are the hosts which current genetic engineering techniques mainly use. Among them, Escherichia coli may be used most easily as the host because of the most advanced elucidation of the genes in its expression system and so forth. However, the use of Escherichia coli does not always result in efficient recovery of heterologous proteins since they remain in cells after production thereof and are exposed to the decomposing and other actions of intracellular enzymes. On the other hand, the use of Bacillus subtilis as the host might result in more efficient recovery of heterologous proteins produced since they are secrected out of cells. However, the expression system in Bacillus subtilis remains unknown in may respects, so that it is not yet always suitable to use *Bacillus subtilis* in commercial production of heterologous proteins.

An object of the invention is to establish a system which is capable of causing secretion of interferon-$\gamma$ into the periplasmic space using gram-negative bacteria.

## SUMMARY OF THE INVENTION

The invention provides a genetic engineering technique by which interferon-$\gamma$ are secreted into the periplasmic space using, as the gene expression elements, expression elements of a gram-positive bacterial strain, in particular, a system containing a *Bacillus natto*-derived $\alpha$-amylase and, as the host, a gram-negative bacterial strain.

## BRIEF DESCRIPTION OF THE DRAWINGS

In the accompaning drawings,

Fig. 1 shows the restriction enzyme map of pGC303,

Fig. 2 shows the scheme of cloning the $\alpha$-amylase gene from chromosomal DNA prepared from *Bacillus natto*,

Figs. 3 and 4 show the process for constructing an expression vector for use in *Escherichia coli*, the numerical figures indicating the respective steps referred to in the detailed description of the invention,

Fig. 5 shows the process for constructing pI$\gamma$amy$_5$ by ligation of pYI7-1 with the IFN-$\gamma$ gene, and

Fig. 6 shows the results of Edman degradation.

## DETAILED DESCRIPTION OF THE INVENTION

(a) Preparation of gene expression elements

The gene expression elements to be used in accordance with the invention is of the gram-positive bacterial strain. The gram-positive bacterial strain is preferably a strain belonging to the genus Bacillus, such as a strain of Bacillus subtilis or Bacillus natto. The term "gene expression elements" includes within the meaning thereof the promoter, SD sequence, signal sequence coding region and so on which are of the gram-positive bacterial strain origin. A preferred example of the gene expression elements is of the $\alpha$-amylase gene. The $\alpha$-amylase gene has been isolated by colleagues to the present inventors from Bacillus natto GC161 deposited at the Fermentation Research Institute under the deposit number FERM BP-539 (cf. European Patent Publication No. 135045; U.S. Ser. No. 633562). A partial nucleotide sequence containing the $\alpha$-amylase promoter and signal sequence coding region is shown in Table 1.

Tabel 1

GTAC AGTCTCGGGC

-200

AGTTTTTTTT ATAGGAACAT TGATTTGTAT TCACTCTGCC AAGTTGTTTT

-150

GATAGAGTGA TTGTGATAAT TTAAAATGTA AGCGTTAACA AAATTCTCCA

-100

GTCTTCACAT CAGCTTGAAA GGAGGAAGCG GAAGAATGAA GTAAGAGGGA

-50 -1

TTTTTGACTC CGAAGTAAGT CTTCAAAAAA TCAAATAAGG AGTGTCAAGA

1 50

ATGTTTGCAA AACGATTCAA AACCTCTTTA CTGCCGTTAT TCGCTGGATT

↳ α-amylase 100

TTTATTGCTG TTTTATTTGG TTCTGGCAGG ACCGGCGGCT GCGAGTGCTG

150

AAACGGCGAA CAAATCGAAT GAGCTTACAG CACCGTCGAT CAAAAGCGGA

200

ACCATTCTTC ATGCATGGAA TTGGTCGTTC AATACGTTAA AACACAATAT

250

GAAGGATATT CATGATGCAG GATATACAGC CATTCAGACA TCTCCGATTA

300

ACCAAGTAAA GGAAGGGAAT CAAGGAGATA AAAGCATGTC GAACTGGTAC

A plasmid containing this gene expression elements is provided under the code pGC303 which is a derivative of the plasmid pGC200 derived from a strain of Bacillus natto deposited at the Fermentation Research Institute under the deposit number FERM BP-645(cf. European Patent Publication No. 135045; U. S. Ser. No. 633562). The restriction enzyme map of pGC303 is shown in Fig. 1.

(b) Preparation of expression vector for use in gram-negative bacteria

The gene expression elements prepared in step (a) is then inserted into such a plasmid as pBR322 derived from Escherichia coli, a gram-negative bacterial species, to creat a plasmid capable of causing expression of interferon-γ in gram-negative bacteria by the action of the gram-positive bacterial gene expression elements.

(c) Ligation with interferon-γ gene

A gene coding for interferon-γ is ligated with the expression vector in gram-negative bacteria as obtained in step (b), at a site downstream from the promoter, SD sequence and signal sequence of said vector, by the known restriction enzyme and ligase treatment process. The ligation product is introduced into an appropriate host.

(d) Transformation

As the host preferred is a gram-negative bacterial strain, in particular a strain of Escherichia coli. The host is transformed by the known method (Cohen, S.N. et al., Proc. Natl. Acad. Sci. USA, 69, 2110 (1972)], for instance.

(e) Cultivation of host

The transformant obtained in step (d) is cultivated on a *per se* known medium suited for growing the host. A preferred example of the medium is the medium used by Palva et al. for growing *Bacillus subtilis* [Gene, 22, 229 (1983)] or a modification thereof. The cultivation is generally conducted at 15-43 ℃ for 3-24 hours, with aeration and/or stirring as necessary.

(f) Recovery of interferon-γ

After cultivation, cells are harvested by a known method (e.g. centrifugation) and the periplasmic fraction is recovered by a known method for recovering proteins secreted into the periplasm [e.g. osmotic shock method [Nossal, N.G. and Heppel, L.A., J. Biol. Chem., 241, 3055 (1966)] ] or a modification thereof. The interferon-γ is recovered from this fraction by a *per se* known method, for example by affinity chromatography and/or fractionation based on solubility differences.

The invention has thus enabled production of interferon-γ in gram-negative bacteria through the use of gene expression elements from gram-positive bacterial strains and has contributed to technology diversification in the field of genetic engineering. On the occasion of the secretion of the interferon-γ into the periplasmic space of gram-negative bacteria, the signal peptide is cleaved off, whereby the protein is obtained in the form of a mature protein free of methionine at the N terminal. Thus, the invention can provide an efficient method of producing interferon-γ using genetic engineering technology.

In the following, the process for producing human interferon-γ (hereinafter, IFN-γ) using α-amylase promoter of the Bacillus natto as a preferred example of gene expression elements of gram-positive bacterial strain and Escherichia coli as a preferred exmaple of the host will be described step by step. It is to be noted, however, this mode of practice is by no means limitative of the scope of the present invention.

(a) Cloning of gene expression elements

A one-liter portion of a overnight culture of Bacillus natto GC161 (deposited at the Fermentation Research Institute under the deposit number FERM BP-539) was centrifuged and the cells obtained were washed. To the cells were added 10 ml of 20 % sucrose-added 50 mM Tris-hydrochloride buffer (pH 8.0) and 100 mg of lysozyme, followed by reaction at 37℃ for 15 minutes. Thereafter, 10 ml of 1% Sarkosyl-1% EDTA solution and 1 ml of Pronase E (100 mg/ml) were added. After

standing at 37℃ for 16 hours, the solution was made up to 35 ml with TES solution (30 mM Tris-HCl, 50 mM NaCl, 5 mM EDTA, pH 8). Thereto were added 35 g of cesium chloride and 1 ml of ethidium bromide (10 mg/ml) and the mixture was centrifuged at 60,000 rpm (Beckman 70.1 Ti rotor) for 16 hours. The chromosomal DNA band was collected, dialyzed against TES solution and treated twice with phenol for purification of the chromosomal DNA.

The chromosomal DNA thus obtained was cleaved with the restriction enzymes *Pvu* II, *Bgl* II, *Hpa* I, *Bam* HI, *Hind* III and so forth. Fragments were extracted from 0.8% agarose gel and ligated with various plasmids, pC194, pE194, pUB110, and pGC200 (deposited at the Fermentation Research Institute under the deposit number FERM BP-645) which is a derivative of pC194, and the ligation products were used for transforming *B. subtilis* ($rec^-$, $amy^-$). After selection based on drug resistance of each plasmid, the transformants selected were inoculated onto starch-added agar plates (L-agar-1% soluble starch). After incubation at 37 ℃ for 2 days, iodine solution (0.1% iodine-1% KI) was added to the agar plates for checking α-amylase activity on the basis of decoloration of iodine. When, among the five restriction enzymes mentioned above, *Hind* III was used for cleaving the chromosomal DNA prepared from Bacillus natto and a fragment of said DNA was inserted into pGC200 at the HindIII site thereof, followed by transformation with the resulting plasmid, there was obtained a strain having α-amylase activity. The chromosomal DNA fragment inserted was a 7 kb fragment. Therefore, the 7 kb DNA containing the α-amylase gene was excised using the restriction enzymes HindIII and RsaI and inserted into the EcoRV-HindIII fragment of pBR322. The plasmid pGC302 thus obtained was then cleaved with SacII, followed by ligation, which gave pGC303 (7.9 kb) (Fig. 2). Transformation was performed in the conventional manner, selection was made based on the ampicillin resistance and the transformants selected were checked for α-amylase activity in the same manner as above, whereupon expression of α-amylase was confirmed. Thus was confirmed the presence of the whole α-amylase gene series. In this manner, the plasmid pGC303 containing the α-amylase promoter, signal sequence coding region and structural gene for α-amylase was obtained.

The DNA sequence of the promoter and signal sequence coding region of α-amylase gene was determined by the Maxam-Gilbert method (Maxam, A. and Gilbert, W., 1979, Methods in Enzymology, 65, 499-560) (Table 1).

(b) Construction of expression vector for use in *Escherichia coli*

An secretion expression vector for use in *Escherichia coli* was constructed from pGC303 and pBR322 in accordance with the procedure shown in Figs. 3 and 4.

Step 1: pGC303 was digested with the restriction enzymes *Ban* III and *Ban* II to give a relatively large DNA fragment containing the promoter and signal sequence coding region of α-amylase gene. The reaction was carried out using the following reaction mixture composition:

| | | |
|---|---|---|
| pGC303 (1.0 μg/μℓ) | 100 | μℓ |
| 10 × *Bcl* I buffer *1 | 20 | μℓ |
| *Ban* II (8 U/μℓ, Toyobo) | 20 | μℓ |
| *Ban* III (5 U/μℓ, Toyobo) | 20 | μℓ |
| $H_2O$ | 40 | μℓ |
| Total | 200 | μℓ |

*1
- 60 mM Tris-HCl (pH 7.4)
- 100 mM $MgCl_2$
- 10 mM dithiothreitol
- 750 mM KCl

The reaction was carried out overnight at 37°C. After completion of the reaction, the whole reaction mixture was applied to 1% agarose gel, followed by electrophoresis. A 960 bp fragment was recovered following adsorption on DEAE-paper. Thereto was added 60μg of tRNA as the carrier for ethanol precipitation. The mixture was extracted with phenol, and the DNA was recovered by ethanol precipitation. The DNA precipitate was dissolved in 90 μl of $H_2O$.

Step 2: The fragment obtained in step 1 was partially digested with AluI using the following reaction mixture composition:

| | |
|---|---|
| DNA fragment solution obtained in step 1 | 50 μl |
| 10×AluI buffer *2 | 20 μl |
| AluI (2 U/μl, Takara Shuzo) | 20 μl |
| $H_2O$ | 110 μl |
| Total | 200 μl |

*2
- 100 mM Tris-HCl (pH 7.5)
- 70 mM $MgCl_2$
- 200 mM NaCl
- 70 mM 2-mercaptoethanol

The reaction was carried out at 37℃ for 5 minutes, and the whole reaction mixture was applied to 5% polyacrylamide gel, followed by electrophoresis. Thereafter, a 621 bp DNA fragment was recovered by electroelution. To the fragment fraction was added 20μg of polyinosic acid was added as the carrier in ethanol precipitation. After extraction with phenol, the fragment was precipitated with ethanol. The DNA precipitate obtained was dissolved in 30 μℓ of $H_2O$.

Step 3: A BamHI linker was ligated with the DNA fragment obtained in step 2 at the AluI end thereof using the following reaction mixture composition:

| | |
|---|---|
| Partial AluI digest fragment obtained in step 2 | 10 μℓ |
| 10×ligation buffer *3 | 1 μℓ |
| BamHI linker *4 | 10 μℓ |
| $T_4$ DNA ligase (175 U/μℓ Takara Shuzo) | 2 μℓ |
| Total | 23 μℓ |

*3
- 660 mM Tris-HCl (pH 7.6)
- 10 mM ATP
- 10 mM spermidine
- 100 mM $MgCl_2$
- 150 mM DTT (dithiothreitol)

*4
- Product of Pharmacia P-L Biochemicals
- Sequence: 5'-d(CCCGGATCCGGG)-3'
- Used after phosphorylation at the 5' end.

The reaction was carried out overnight at 4 ℃, followed by extraction with phenol and precipitation with ethanol. The DNA precipitate was dissolved in 42 μℓ of $H_2O$.

Step 4: The DNA fragment obtained in step 3 was digested with BamHI using the following reaction mixture composition:

| | |
|---|---|
| DNA obtained in step 3 | 42 μl |
| 10×BamHI buffer *5 | 5 μl |
| BamHI (12 U/μl, Takara Shuzo) | 3 μl |
| Total | 50 μl |

*5
- 100 mM Tris-HCl (pH 8.0)
- 70 mM $MgCl_2$
- 1 M NaCl
- 20 mM 2-mercaptoethanol

The reaction was carried out overnight at 37℃ and the whole reaction mixture was applied to 5% polyacrylamide gel for electrophoresis. After electrophoresis, a 533 bp fragment was recovered by electroelution, followed by extraction with phenol and precipitation with ethanol. The DNA precipitate recovered was dissolved in 50 μl of $H_2O$.

Step 5: For use as a vector for cloning the DNA fragment (both ends being BamHI ends) obtained in step 4, pBR322 was digested with BamHI overnight at 37 ℃ using the following reaction mixture composition:

| | |
|---|---|
| pBR322 (1.0 μg/μl) | 25 μl |
| 10×BamHI buffer(mentioned above) | 5 μl |
| BamHI (10 U/μl, Takara Shuzo) | 3 μl |
| $H_2O$ | 17 μl |
| Total | 50 μl |

and then the phosphoric acid residue at the 5' end was eliminated by using bacterial alkaline phosphatase(BAP).

After reaction, DNA was recovered by precipitation with ethanol. The DNA was dissolved in 180 μℓ of $H_2O$.

To prevent self ligation of the vector (pBR322), this was treated with bacterial alkaline phosphatase (BAP) at 65 ℃ for 40 minutes for eliminating the phosphoric acid residue at the 5' end. The reaction mixture composition was as follows:

| | | |
|---|---|---|
| BamHI-digested pBR322 | | 180 μℓ |
| 10×BAP buffer *6 | | 20 μℓ |
| BAP (0.45 U/μℓ, Takara Shuzo) | | 2 μℓ |
| | Total | 202 μℓ |

*6
- 100 mM Tris-HCl (pH 7.5)
- 70 mM $MgCl_2$
- 600 mM NaCl

After reaction, phenol extraction was repeated three times and DNA was recovered by precipitation with ethanol. The DNA precipitate was dissolved in 25 μℓ of $H_2O$.

Step 6: The DNA fragment obtained in step 4 and the vector obtained in step 5 were ligated together by overnight reaction at 4 ℃. The reaction mixture composition was as follows:

| | |
|---|---|
| DNA fragment from step 4 | 10 μℓ |
| BamHI-digested and BAP-treated pBR322 | 5 μℓ |
| 10× ligation buffer *7 | 4 μℓ |
| $T_4$ DNA ligase (175 U/μℓ, Takara Shuzo) | 1 μℓ |
| Total | 20 μℓ |

*7
- 660 mM Tris-HCl(pH 7.6)
- 50 mM $MgCl_2$
- 50 mM DTT(dithiothreitol)
- 10 mM ATP

After reaction, the whole reaction mixture was used for transformation of E. coli RR1 by the method of Cohen et al. As a result, there were obtained 6,500 colonies, 200 of which were checked for sensitivity to ampicillin (Ap) and to tetracycline (Tc). Fourteen strains were found to be resistant to Ap ($Ap^r$) and susceptible to Tc ($Tc^s$). Using these 14 strains, a plasmid was prepared in large quantities [according to Clewell, D. B. and Helinski, D. R., Proceedings of the National Academy of Sciences USA, 62, 1159 (1969)] by the alkaline extraction method [Birnboim, H. C., and Doly, J., Nucleic Acids Research, 7, 1513 (1979)]. This plasmid was named pYI5-1.

Step 7: pYI5-1 is inconvenient for inserting

the IFN-γ gene since it has two BamHI sites. Therefore, for causing one BamHI site (upstream from the α-amylase promoter) to disappear, pYI5-1 was first digested with AvaI at 37°C overnight. The reaction mixture composition used was as follows:

| | |
|---|---|
| pYI5-1 (1.43 μg/μℓ) | 14 μℓ (20 μg) |
| 10×AvaI buffer *8 | 5 μℓ |
| AvaI (8 U/μℓ, Takara shuzo) | 3 μℓ |
| $H_2O$ | 28 μℓ |
| Total | 50 μℓ |

*8
- 100 mM Tris-HCl (pH 8.0)
- 70 mM $MgCl_2$
- 600 mM NaCl
- 70 mM 2-mercaptoethanol

After reaction, the whole reaction mixture was applied to 1% agarose gel and electrophoresis was performed. A DNA fragment of about 3.5 kbp was caused to be adsorbed on DEAE-paper and then recovered. As the carrier to serve in precipitation with ethanol, there was added 20 μg of tRNA. Following extraction with phenol, DNA was recovered by precipitation with ethanol. The DNA recovered was dissolved in 50 μℓ of $H_2O$.

Step 8: The AvaI-digested pYI5-1 obtained in step 7 was cyclized by allowing self ligation to take place

at 4℃ overnight. The reaction mixture composition used was as follows:

| | |
|---|---|
| AvaI-digested pYI5-1 obtained in step 7 | 2 μℓ |
| 10×ligation buffer (same in composition as in step 6) | 10 μℓ |
| $T_4$ DNA ligase (175 U/μℓ, Takara Shuzo) | 1 μℓ |
| $H_2O$ | 87 μℓ |
| Total | 100 μℓ |

A 25 μℓ portion of the reaction mixture (100 μℓ) was used for transformation of E. coli RR1, whereby 18 ampicillin-resistant ($Ap^r$) colonies were obtained. These colonies were subjected to plasmid extraction by the alkline extraction method and the plasmids extracted were examined for their structure using various restriction enzymes. As a result, 14 colonies were found to have a plasmid having the structure of pYI6-1 given in Fig. 3. One of these strains was used for large-quantity plasmid preparation and the plasmid obtained was named pYI6-1.

Step 9: pYI6-1 was digested with the restriction enzyme HpaII to give a DNA fragment containing the α-amylase promoter and part of the signal sequence. For the digestion treatment, the following reaction mixture composition was used:

| | |
|---|---|
| pYI6-1 (0.53 μg/μℓ) | 187 μℓ |
| 10×*Hpa* II buffer *9 | 30 μℓ |
| *Hpa* II (6 U/μℓ, Toyobo) | 40 μℓ |
| $H_2O$ | 43 μℓ |
| Total | 300 μℓ |

*9
- 100 mM Tris-HCl (pH 7.5)
- 70 mM $MgCl_2$
- 70 mM 2-mercaptoethanol

The reaction was carried out at 37℃ for 5 hours. Thereafter, the whole reaction mixture was applied to a 5% polyacrylamide gel, followed by electrophoresis. A 348 bp DNA fragment was recovered by electroelution. To the fragment recovered was added 40 μℓ of tRNA as a carrier in ethanol precipitation. After extraction with phenol, the DNA was precipitated with ethanol, and the DNA precipitate obtained was dissolved in 60 μℓ of $H_2O$.

Step 10: The DNA fragment obtained in step 9 was rendered blunt-ended using DNA polymerase I in a reaction mixture having the following composition:

| | |
|---|---|
| *Hpa* II fragment obtained in step 9 | 24 μℓ |
| 10×DNA polymerase I buffer *10 | 15 μℓ |
| dNTP *11 | 6 μℓ |
| DNA polymerase I, large fragment (6 U/μℓ, Toyobo) | 4 μℓ |
| $H_2O$ | 101 μℓ |
| Total | 150 μℓ |

*10 ┌ 500 mM Tris-HCl (pH 7.2)
│ 100 mM $MgCl_2$
└ 1 mM dithiothreitol

*11 Aqueous solution containing dATP, dTTP, dGTP and dCTP each in a concentration of 2 mM

The reaction was carried out at 20℃ for 45 minutes. After reaction, 120 μl of 5 M ammonium acetate and 540 μl of cold ethanol were added for ethanol precipitation. The DNA precipitate obtained was dissolved in 20 μl of $H_2O$.

Step 11: A mixture of a BamHI linker and a PstI linker was allowed to add to both ends of the DNA fragment obtained in step 10 using the following reaction mixture composition:

| | |
|---|---|
| DNA fragment obtained in step 10 | 10 μl |
| 10 × ligation buffer (mentioned above) | 2 μl |
| BamHI linker (0.2 μg/μl, Nippon Zeon)*12 | 10 μl |
| PstI linker (1.0 μg/μl, Takara Shuzo) *13 | 3 μl |
| $T_4$ DNA ligase (175 U/μl, Takara Shuzo) | 2 μl |
| Total | 27 μl |

*12 the sequence 5'-d(GCAGGATCCTGC)-3'; used after addition of phosphryl group at the 5'-end.

*13 the sequence 5'-(d(pGCTGCAGC)-3', with a

phosphoryl group already added in advance at the 5'-end.

The reaction was carried out at 4°C overnight. Thereafter, extraction with phenol was performed, followed by precipitation with ethanol. The DNA precipitate obtained was dissolved in 37 μℓ of $H_2O$.

Step 12: The DNA fragment obtained in step 11 was digested with BamHI and AvaI using the following reaction mixture composition:

| | |
|---|---|
| DNA fragment obtained in step 11 | 37 μℓ |
| 10×BamHI buffer (mentioned above) | 5 μℓ |
| BamHI (12 U/μℓ, Takara Shuzo) | 3 μℓ |
| AvaI (8 U/μℓ, Takara Shuzo) | 5 μℓ |
| Total | 50 μℓ |

The reaction was carried out at 37°C overnight and, then, the whole reaction mixture was applied to a 5% polyacrylamide gel, followed by electrophoresis. Thereafter, a DNA fragment of about 300 bp was recovered by electroelution. After extraction with phenol, the DNA was precipitated with ethanol. On that occasion, 40 μg of tRNA was added as a carrier. The DNA precipitate recovered was dissolved in 50 μℓ of $H_2O$.

Step 13: pBR322, chosen as a vector for cloning the DNA fragment obtained in step 12 (both ends being AvaI and BamHI), was digested with BamHI and AvaI using

the following reaction mixture composition:

| | |
|---|---|
| pBR322 (1.0 μg/μℓ) | 25 μℓ |
| 10 × BamHI buffer | 5 μℓ |
| BamHI (12 U/μℓ, Takara Shuzo) | 3 μℓ |
| AvaI (8 U/μℓ, Takara Shuzo) | 6 μℓ |
| $H_2O$ | 11 μℓ |
| Total | 50 μℓ |

The reaction was carried out at 37℃ overnight. Then, for preventing self ligation of the vector (pBR322), the 5'-end phosphoryl groups were removed using bacterial alkaline phosphatase (BAP). Thus, 2 μℓ of BAP (0.45 U/μℓ, Takara Shuzo) was added to the above reaction mixture and the reaction was carried out at 65℃ for 40 minutes. After reaction, the whole reaction mixture was applied to a 1% agarose gel, and electrophoresis was conducted. A 3,300 bp DNA fragment was allowed to be adsorbed on a DEAE-paper, and then recovered. As a carrier in ethanol precipitation, 40 μg of tRNA was added. The mixture was extracted with phenol and the DNA was recovered by precipitation with ethanol. The DNA precipitate thus obtained was dissolved in 90 μℓ of $H_2O$.

Step 14: The DNA fragment obtained in step 12 and the voctor obtained in step 13 were ligated together using the following reaction mixture composition:

| | |
|---|---|
| DNA fragment from step 12 | 10 μℓ |
| Vector DNA from step 13 | 5 μℓ |
| 10× ligation buffer (mentioned above) | 4 μℓ |
| $T_4$ DNA ligase (175 U/μℓ, Takara Shuzo) | 1 μℓ |
| $H_2O$ | 20 μℓ |
| Total | 40 μℓ |

The reaction was carried out at 4 ℃ overnight. After completion of the reaction, the whole reaction mixture was used for transformation of E. coli RR1 by the method of Cohen et al. As a result, 3,500 colonies were obtained. Among them, 48 strains were used for plasmid preparation by the alkaline extraction method, and the structure of each plasmid was examined using various restriction enzymes. As a result, one strain was found to carry a plasmid of the structure of pYI7-1 as shown in Fig. 4, and this plasmid was named pYI7-1 and used in the subsequent steps.

The plasmid pYI7-1 thus obtained has a BamHI site downstream from the signal sequence (31 amino acids) for the Bacillus natto α-amylase gene and, further downstream therefrom, one each of EcoRI, ClaI, HindIII and EcoRV sites. By making use of these sites, it is possible to insert into the plasmid a heterologous gene such as an IFN-γ gene. Thus, a DNA fragment having a BamHI site upstream from a gene coding for a heterologous protein such as IFN-γ and, downstream therefrom, a flush end site such as an EcoRI, ClaI, HindIII and/or further EcoRV site can be inserted into said plasmid.

(c) Ligation of pYI7-1 with IFN-γ gene (construction of pIγamy$_5$)

Utilizing pYI7-1 a DNA fragment containing an IFN-γ gene inserted into pYI7-1 between the BamHI site and the EcoRV site thereof to give pIγamy$_5$ for expression of the IFN-γ gene and secretion of IFN-γ. The construction of pIγamy$_5$ is schematically shown in Fig. 5.

Step 1: pYI7-1 was digested with BamHI and EcoRV using the following reaction mixture composition:

| | |
|---|---|
| pYI7-1 (1.0 μg/μℓ) | 50 μℓ |
| 10×BamHI buffer (mentioned above) | 15 μℓ |
| BamHI (12 U/μℓ, Takara Shuzo) | 10 μℓ |
| EcoRV (5 U/μℓ, Takara Shuzo) | 20 μℓ |
| $H_2O$ | 55 μℓ |
| Total | 150 μℓ |

The reaction was carried at 37℃ overnight. Then, to prevent self ligation of the vector (pYI7-1), the 5'-end phosphoryl groups were removed using bacterial alkaline phosphatase (BAP). Thus, 2 μℓ of BAP (0.45 U/μℓ, Takara Shuzo) was added to the above reaction mixture and the reaction was carried out at 65℃ for 40 minutes. Thereafter, the whole reaction mixture was applied to a 1% agarose gel and electrophoresis was performed. Then, a DNA fragment of about 3.7 Kbp was recovered following adsorption thereof on a DEAE-paper. Thereto was added 40 μg of tRNA as a carrier in ethanol precipitation. After extraction of the mixture with phenol, the DNA was recovered by precipitation with ethanol. The DNA precipitate obtained was dissolved in 50 μℓ of $H_2O$.

Step 2: A genetically engineered, IFN-γ gene-containing plasmid, pYS61, having the construction shown in Fig. 5 was digested with NdeI and HindIII using the following reaction mixture composition:

| | |
|---|---|
| pYS61 (1.2 μg/μℓ) | 42 μℓ |
| 10×NdeI buffer *14 | 10 μℓ |
| NdeI (8 U/μℓ, NEB) | 10 μℓ |
| HindIII (8 U/μℓ, Takara Shuzo) | 10 μℓ |
| $H_2O$ | 28 μℓ |
| Total | 100 μℓ |

*14
- 100 mM Tris-HCl (pH 7.8)
- 1.5 M NaCl
- 70 mM $MgCl_2$
- 60 mM 2-mercaptoethanol

The reaction was carried out at 37°C overnight. Thereafter, the whole reaction mixture was applied to a 1% agarose gel and electrophoresis was conducted. A DNA fragment of about 700 bp was adsorbed on a DEAE-paper and then recovered. After addition of 40 μg of tRNA as a carrier in ethanol precipitation, the mixture was extracted with phenol. Then, the DNA was recovered by precipitation with ethanol. The DNA obtained was dissolved in 50 μℓ of $H_2O$. The DNA fragment obtained in this step contains approximately the whole of the IFN-γ gene and the trp terminator but is lacking in that portion which code for the three terminal amino acids from the N-terminal of IFN-γ. However, the DNA base sequence corresponding to these three amino acids can be reconstructed when said fragment is subsequently inserted into pYI7-1 following BamHI linker addition to said fragment.

Step 3: The DNA fragment obtained in step 2 was rendered blunt-ended using DNA polymerase I and the following reaction mixture composition:

| | |
|---|---|
| DNA fragment obtained in step 2 | 25 μℓ |
| 10 × DNA polymerase I buffer (mentioned above) | 15 μℓ |
| dNTP (each 2 mM; mentioned above) | 6 μℓ |
| DNA polymerase I, large fragment (6 U/μℓ, Toyobo) | 4 μℓ |
| $H_2O$ | 100 μℓ |
| Total | 150 μℓ |

The reaction was carried out at 20℃ for 45 minutes. Thereafter, 120 μℓ of 5 M ammonium acetate and 540 μℓ of cold ethanol were added to thereby cause ethanol precipitation. The DNA precipitate obtained was dissolved in 20 μℓ of $H_2O$.

Step 4: A BamHI linker was connected to both ends of the DNA fragment obtained in step 3 using the following reaction mixture composition:

| | |
|---|---|
| DNA fragment obtained in step 3 | 10 μℓ |
| 10 × ligation buffer (mentioned above) | 2 μℓ |
| BamHI linker (1.0 μg/μℓ, Takara Shuzo) *15 | 2 μℓ |
| $T_4$ DNA ligase (175 U/μℓ, Takara Shuzo) | 2 μℓ |
| $H_2O$ | 4 μℓ |
| Total | 20 μℓ |

*15 The sequence 5'-d(pCGGATCCG)-3';having, at the 5'-end, a phosphoryl group added beforehand

The reaction was carried out at 4℃ overnight. Thereafter, following extraction with phenol, precipitation was effected by addition of ethanol. The DNA precipitate obtained was dissolved in 30 μℓ of $H_2O$.

Step 5: The DNA fragment obtained in step 4 was digested with BamHI and HincII using the following reaction mixture composition:

| | | |
|---|---|---|
| DNA fragment obtained in step 4 | | 30 μℓ |
| 10×BamHI buffer (mentioned above) | | 5 μℓ |
| BamHI (12 U/μℓ, Takara Shuzo) | | 3 μℓ |
| HincII (6 U/μℓ, Takara Shuzo) | | 6 μℓ |
| $H_2O$ | | 6 μℓ |
| | Total | 50 μℓ |

The reaction was carried out at 37℃ overnight and, then, the whole reaction mixture was applied to a 5% polyacrylamide gel, followed by electrophoresis. After migration, a fragment of about 700 bp was recovered by electroelution. After extraction with phenol, 30 μg of tRNA was added as a carrier and precipitation was caused with ethanol. The DNA precipitate thus recovered was dissolved in 25 μℓ of $H_2O$.

Step 6: The vector prepared in step 1 and the IFN-γ gene-containing DNA fragment prepared in step 5 were ligated together using the following reaction mixture composition:

| | | |
|---|---|---|
| Vector fragment obtained in step 1 | | 2 μℓ |
| DNA fragment obtained in step 5 | | 5 μℓ |
| 10×ligation buffer (mentioned above) | | 3 μℓ |
| $T_4$ DNA ligase (175 U/μℓ, Takara Shuzo) | | 2 μℓ |
| $H_2O$ | | 18 μℓ |
| | Total | 30 μℓ |

The reaction was carried out at 4℃ overnight.

(d) Transformation

After the reaction in the above step 6, the whole reaction mixture was used for transformation of E. coli HB101. As a result, 24 $Ap^r$ transformants were obtained. These strains were subjected to plasmid extraction by the alkaline method and the plasmids extracted were examined for their structure using various restriction enzymes. 22 Strains were found to have pIγ$amy_5$ shown in Fig. 5. One of these strains (pIγ$amy_5$-carrying E. coli HB101) was checked for its ability to produce IFN-γ.

(e) Production of IFN-γ

The pIγ$amy_5$-carrying strain of E. coli HB101 was grown in a 2-liter erlenmeyer flask with shaking at 37℃ using 500 ml of a medium (specified below) according to Palva et al. [Gene, 22, 229 (1983)], with checking at timed intervals for bacterial growth in terms of measured $A_{650}$ values.

| | |
|---|---|
| Yeast extract (Difco) | 10 g/liter |
| Bactotryptone (Difco) | 20 g/liter |
| NaCl | 10 g/liter |
| $(NH_4)_2SO_4$ | 1 g/liter |
| $K_2HPO_4$ | 7 g/liter |
| $KH_2PO_4$ | 2 g/liter |
| Ampicillin | 25 mg/liter |

(f) Recovery of IFN-γ

The culture obtained in (e) was sampled (20 ml) at 3-hour intervals and the samples were each fractionated according to the flowchart given later herein. The culture supernatant, cell washings (1) and (2), periplasmic fraction and cell extract were assayed for IFN-γ titer by the RPHA method. At 12 hours after start of cultivation, the IFN-γ potencies thus measured are shown in Table 2.

Table 2

| IFN-γ titer (IU/liter of medium) | |
|---|---|
| Culture supernatant | $1.88 \times 10^4$ |
| Cell washings | $2.65 \times 10^4$ |
| Cell extract | $2.65 \times 10^6$ |
| Periplasmic fraction | $3.13 \times 10^6$ |

Then, the periplasmic fractions and cell extract fractions collected at 3-hour intervals over the period of hour 6 to hour 24 were assayed for IFN-γ titer. The results thus obtained are shown in Table 3. As is evident from the data given in Table 3, the yield increased with the growth of bacteria and became constant at 15 hours after start of cultivation. Not less than 50% of intracellular IFN-γ was found secreted in the periplasmic space.

Table 3

IFN-γ titer (IU/liter of medium)

| Cultivation period | Periplasmic fraction | Cell extract | $A_{650}$ |
|---|---|---|---|
| 6 | $5.22 \times 10^5$ | $6.63 \times 10^5$ | 3.70 |
| 9 | $1.04 \times 10^6$ | $1.33 \times 10^6$ | 7.15 |
| 12 | $3.13 \times 10^6$ | $2.65 \times 10^6$ | 9.40 |
| 15 | $3.13 \times 10^6$ | $3.98 \times 10^6$ | 10.90 |
| 18 | $3.13 \times 10^6$ | $3.98 \times 10^6$ | 11.00 |
| 21 | $3.13 \times 10^6$ | $3.98 \times 10^6$ | 11.01 |
| 24 | $3.13 \times 10^6$ | $3.98 \times 10^6$ | 11.03 |

(g) Examination of IFN-γ secreted into periplasmic fraction for some characteristics

The pIγamy$_s$-carrying strain E. coli HB101 was shake-cultured at 37℃ in a 100-ml erlenmeyer flask using 20 ml of a medium according to Palva et al.(mentioned above). Nine hours after start of cultivation, when $A_{650}$=6.50, 200 μCi of [$^3$H] glutamine or [$^3$H] lysine (Amersham) was added and the shake culture was continued for additional 15 minutes. Thereafter, the erlenmeyer flask was cooled with ice to thereby terminate the labelling. Cells were harvested by centrifugation and a periplasmic fraction was prepared accoding to the flowchart given later herein. The periplasmic fraction obtained was applied to a column packed with 2 ml of anti-IFN-γ monoclonal antibody Sepharose to thereby cause adsorption of IFN-γ. The column was washed with 0.1-1.0 M NaCl for removal of impurities. Then, 3.5 M KSCN was poured into the column to cause elution of IFN-γ. The KSCN was removed by dialysis and the dialysate was concenterated to about 200 μℓ by ultrafiltration. Then, a 25 μℓ portion of the thus-purified IFN-γ solution was subjected to SDS-polyacrylamide gel electrophoresis. After fixation of the gel, sensitization and drying, autoradiography was performed. As a result, a single band was observed in the neighborhood of a molecular weight of about 18,000.

In the next place, a 100 μℓ portion of the purified IFN-γ was subjected to Edman degraddation using an Applied Biosystems model 470A protein sequencer. Each fraction from the N-terminal as obtained was measured for radio-activity using a liquid scintillation counter. The results are shown in Fig. 6. As is evident from the results, in the case of labelling with ($^{3}H$) glutamine, $^{3}H$ uptake was observed in the first cycle whereas in the case of labelling with ($^{3}H$) lysine, $^{3}H$ uptake was noted in the 6th, 12th and 13th cycles.

This suggests that the IFN-γ secreted into the periplasmic fraction has the following amino acid sequence in the neighborhood of its N-terminal:

1 5 10
Glu-X-X-X-X-Lys-X-X-X-X-X-Lys-Lys- (X: unknown).

The above sequence is in agreement with the amino acid sequence of mature IFN-γ which starts with Gln shown in Table 4. Thus, it was confirmed that the IFN-γ secreted into the periplasmic space has the same amino acid sequence as that of native IFN-γ which is free of Met at the N-terminal.

Table 4

α-amylase signal sequence and base sequence of linking portion of IFN-γ gene in pIγamy5 and corresponding amino acid sequence

| | | | | | | | | | | | | | | | | | | | |
|---|---|---|---|---|---|---|---|---|---|---|---|---|---|---|---|---|---|---|---|
| | 27 | 28 | 29 | 30 | 31 | 1 | 2 | 3 | 4 | 5 | 6 | 7 | 8 | 9 | 10 | 11 | 12 | 13 | |
| | Gly | Pro | Ala | Ala | Ala | Gln | Asp | Pro | Tyr | Val | Lys | Glu | Ala | Glu | Asn | Leu | Lys | Lys | |
| --- | GGA | CCG | GCT | GCA | GCG | CAG | GAT | CCG | TAT | GTA | AAA | GAA | GCA | GAA | AAC | CTT | AAG | AAA | --- |
| --- | CCT | GGC | CGA | CGT | CGC | GTC | CTA | GGC | ATA | CAT | TTT | CTT | CGT | CTT | TTG | GAA | TTC | TTT | |

α-amylase signal sequence ⟶ | IFN-γ ⟶

Flowchart illustrating the process for recovering IFN-$\alpha_2$

20 ml of culture

Centrifugation (8,000 rpm, 4℃, 10 minutes)

Cell pellet — Supernatant (culture supernatant)

Suspended in 5 ml of cold aqueous solution containing 10 mM Tris-HCl (pH 8.0) and 30 mM NaCl

Centrifugation (6,000 rpm, 4℃, 5 minutes)

Cell pellet — Supernatant (cell washings) (1)

Suspended in 5 ml of aqueous solution containing 20% sucrose and 30 mM Tris-HCl (pH 8.0)

Addition of 10 μℓ of 0.5 M EDTA (pH 8.0)

(final concentration 1 mM EDTA)

Shaking at room temperature for 10 minutes

Centrifugation (6,000 rpm, 4℃, 5 minutes)

(to be continued)

Cell pellet → Supernatant (cell washings) (2)

Cell pellet

Suspended in 5 ml of cold distilled water

Shaking on an ice bath for 10 minutes

Centrifugation (6,000 rpm, 4℃, 5 minutes)

Cell pellet → Supernatant (periplasmic fraction)

Suspended in 1.5 ml of lysis buffer*

Allowed to stand at 0℃ for 30 minutes

Five repetitions of freezing-thawing cycle

Centrifugation (18,000 rpm, 4℃, 90 minutes)

Precipitate → Supernatant (cell extract)

* 50 mM Tris-HCl (pH 8.0), 30 mM NaCl, 6 mM EDTA, 10 μg/ml phenylmethylsulfonyl fluoride, 1 mg/ml lysozyme

What is claimed is:

1. A method of producing a interferon-$\gamma$ which comprises using, as the gene expression elements, a expression elements containing an $\alpha$-amylase promoter region derived from a gram-positive bacterial strain and, as the host, a gram-negative bacterial strain.

2. The method of claim 1, wherein the gram-positive bacterial strain is a strain belonging to the genus _Bacillus_.

3. The method of Claim 2, wherein the strain belonging to the genues _Bacillus_ is a strain of the species _Bacillus natto_.

4. The method of any of claims 1-4, wherein the gram-negative bacterial strain is a strain of _Escherichia coli_.

0190686

1/6

FIG 1

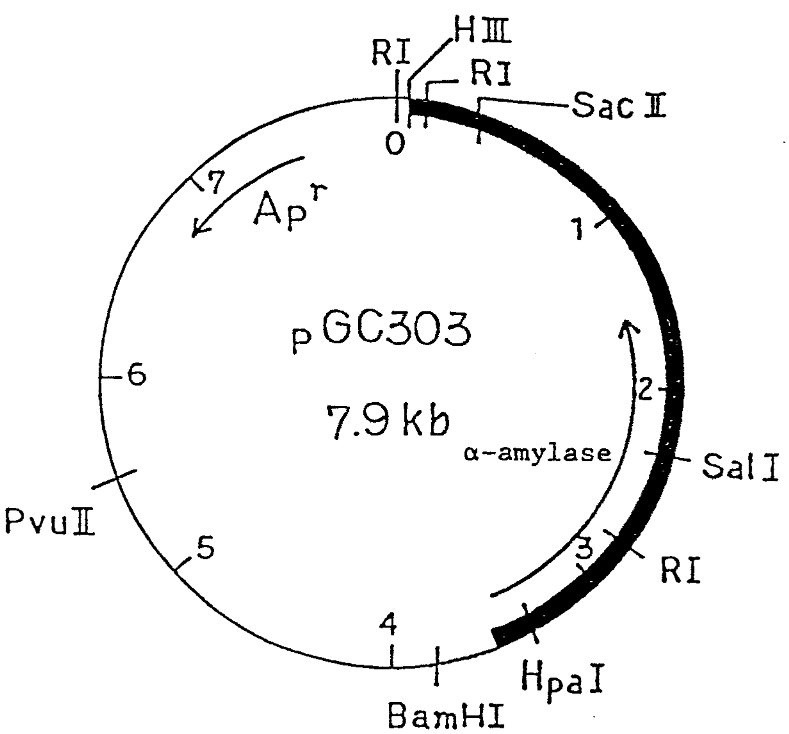

FIG 2

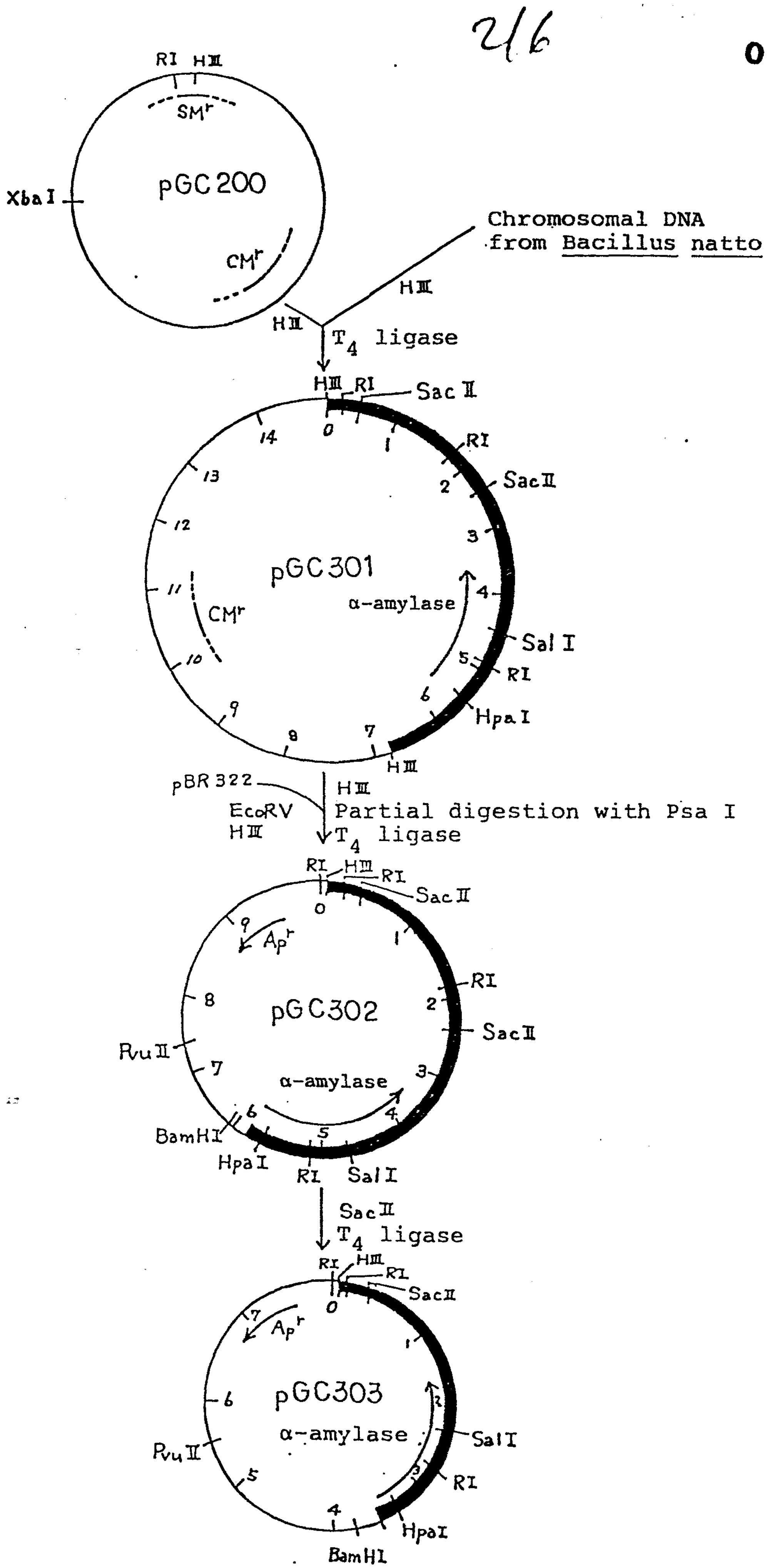
RI HIII
SMr
XbaI
pGC200
CMr
Chromosomal DNA
from Bacillus natto
HIII
HIII
T4 ligase
HIII RI Sac II
RI
SacII
pGC301
CMr
α-amylase
Sal I
RI
HpaI
HIII
pBR322
EcoRV
HIII
HIII
Partial digestion with Psa I
T4 ligase
RI HIII RI
SacII
Apr
pGC302
RI
SacII
PvuII
α-amylase
BamHI
HpaI
RI
SalI
SacII
T4 ligase
RI HIII RI
SacII
Apr
pGC303
α-amylase
SalI
PvuII
RI
HpaI
BamHI

3/6

0190686

FIG 3

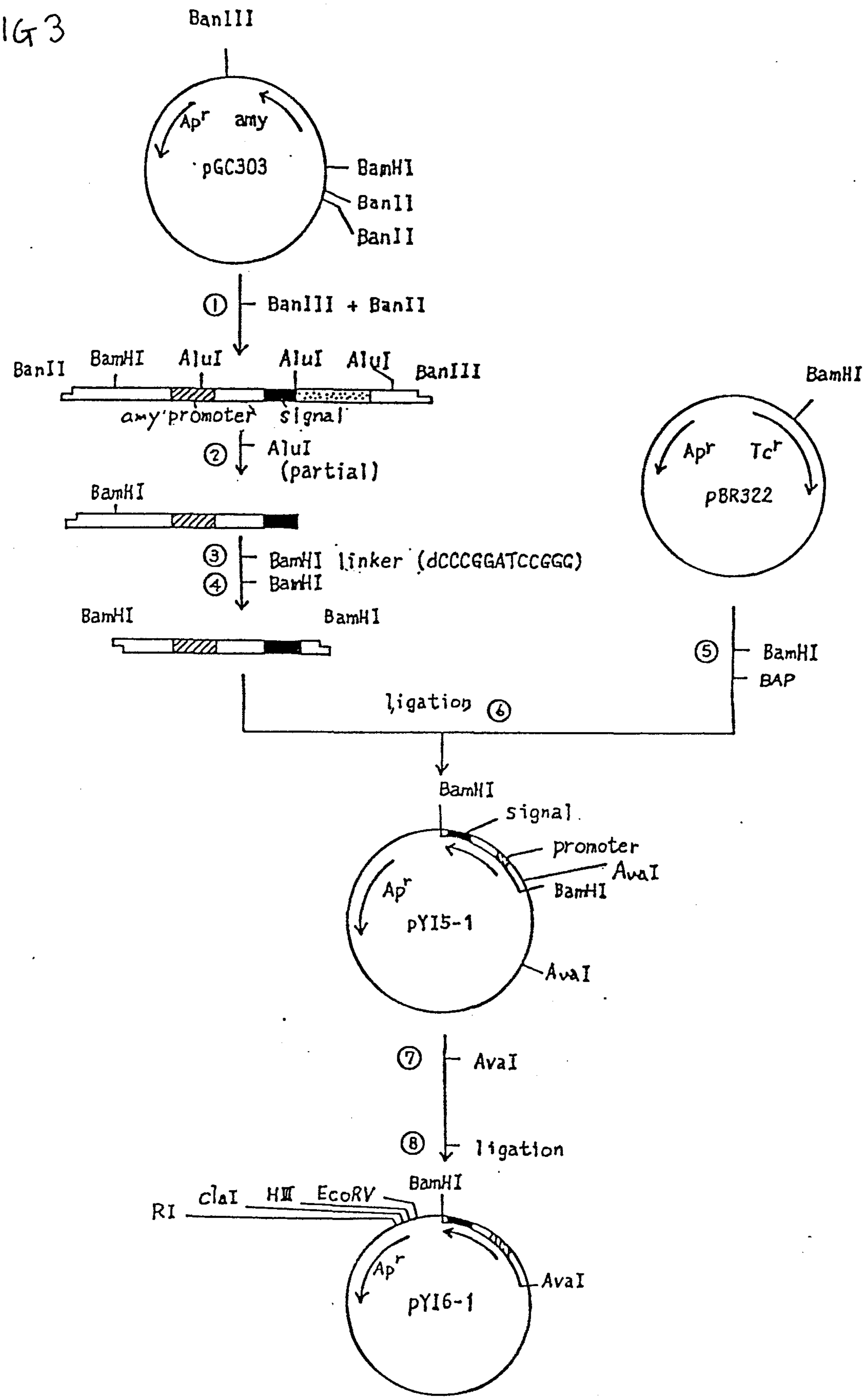

Fig 4

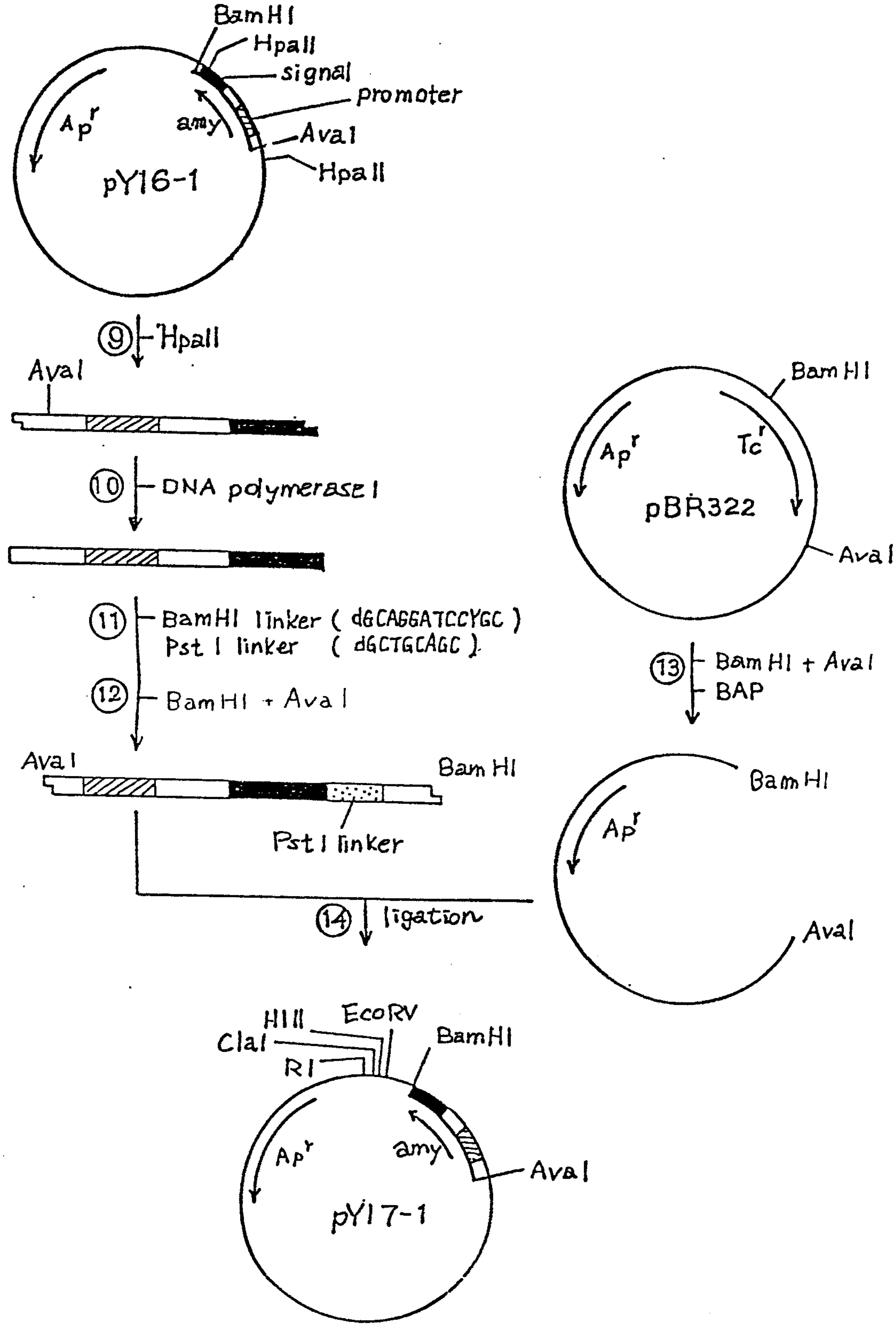
BamHI
HpaII
signal
promoter
amy
Ap^r
AvaI
HpaII
pY16-1
⑨ HpaII
AvaI
⑩ DNA polymerase I
⑪ BamHI linker ( dGCAGGATCCYGC )
Pst I linker ( dGCTGCAGC )
⑫ BamHI + AvaI
AvaI
BamHI
PstI linker
BamHI
Ap^r
Tc^r
pBR322
AvaI
⑬ BamHI + AvaI
BAP
BamHI
Ap^r
AvaI
⑭ ligation
HIII
EcoRV
ClaI
RI
BamHI
Ap^r
amy
AvaI
pY17-1

Fig 5

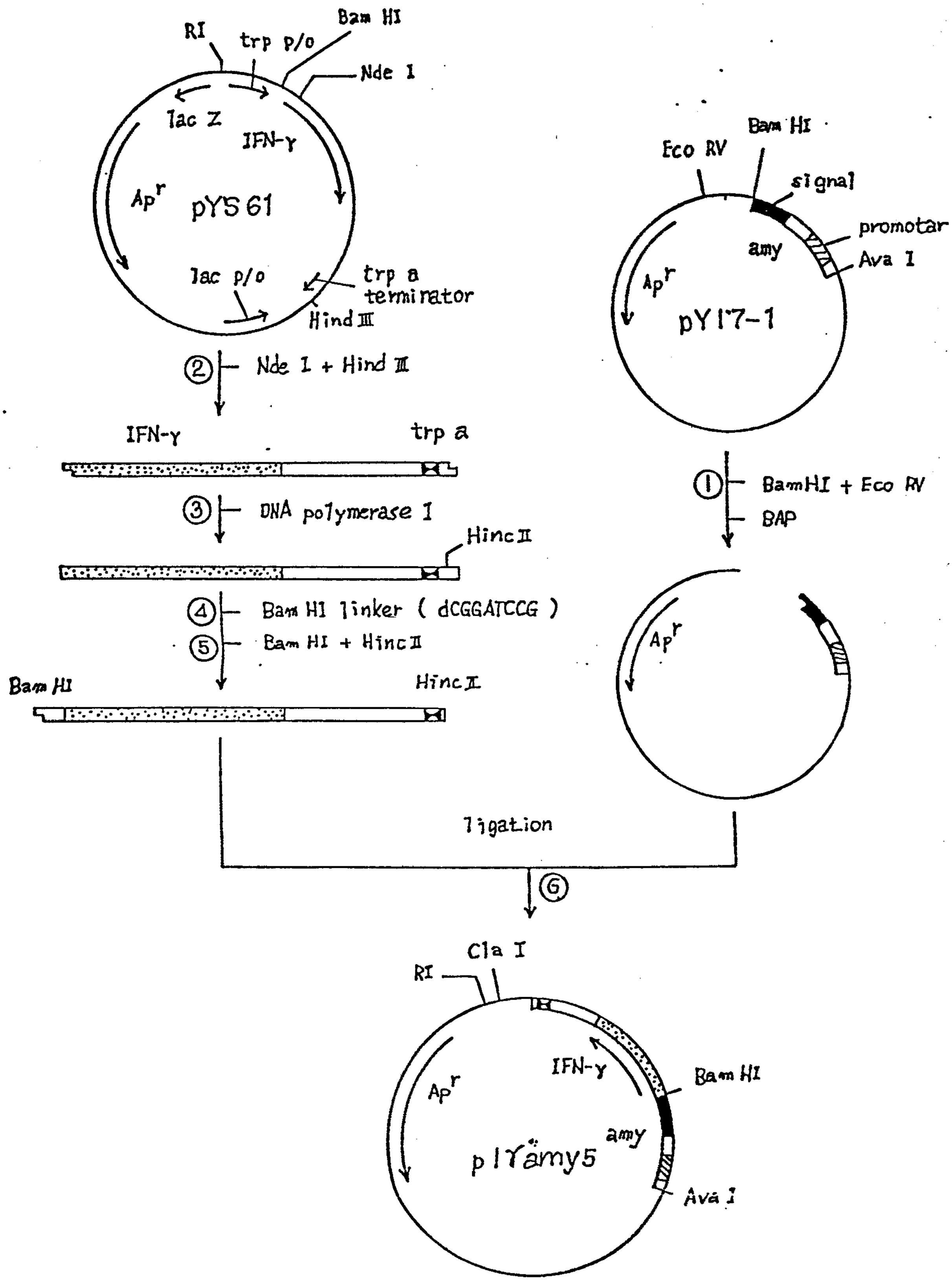
RI
trp p/o
Bam HI
Nde I
lac Z
IFN-γ
Ap^r
pYS 61
lac p/o
trp a terminator
Hind III
② Nde I + Hind III
IFN-γ
trp a
③ DNA polymerase I
Hinc II
④ Bam HI linker ( dCGGATCCG )
⑤ Bam HI + Hinc II
Bam HI
Hinc II
Bam HI
Eco RV
signal
promotar
amy
Ava I
Ap^r
pY 17-1
① BamHI + Eco RV
BAP
Ap^r
ligation
⑥
Cla I
RI
IFN-γ
Bam HI
Ap^r
amy
p I γ amy5
Ava I

Fig.6

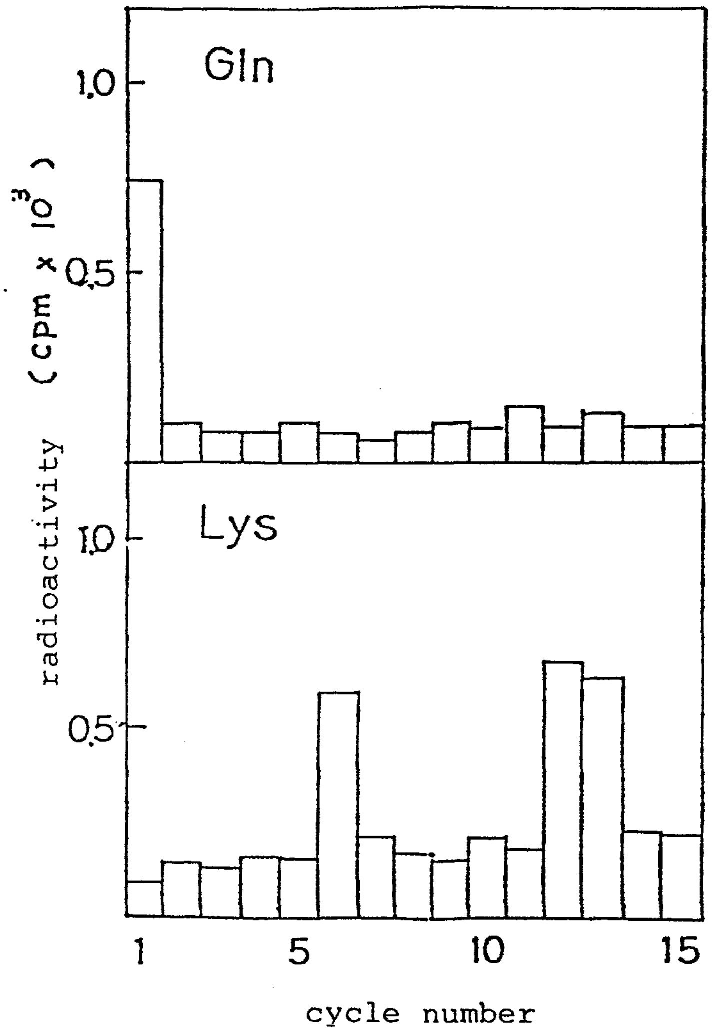
Gln
Lys
1.0
0.5
1.0
0.5
radioactivity (cpm x 10³)
1
5
10
15
cycle number